# EUROPEAN PATENT SPECIFICATION

(11) **EP 2 694 561 B1**
(45) Date of publication and mention of the grant of the patent: **14.10.2015**
(21) Application number: 12713944.2
(22) Date of filing: 03.04.2012
(51) Int. Cl.: C08F 222/00, C09J 4/00, A61L 24/06, C08F 222/32, C08L 35/04

(54) **NOVEL ADHESIVE COMPOSITIONS AND USES THEREOF**
NEUE KLEBEZUSAMMENSETZUNGEN UND DEREN VERWENDUNGEN
NOUVELLES COMPOSITIONS ADHÉSIVES ET LEURS UTILISATIONS

(30) Priority: 04.04.2011 EP 11382094
(43) Date of publication of application: 12.02.2014
(73) Proprietor: Bioadhesives Medtech Solutions, S.L., 03206 Elche (Alicante) (ES)
(72) Inventor: MARTÍN MARTÍNEZ, José Miguel, E-03206 Elche (Alicante) (ES); MARTÍNEZ PAÍNO, Manuel, E-03206 Elche (Alicante) (ES); DOMÍNGUEZ ARRIBAS, Luis Esteban, E-03206 Elche (Alicante) (ES); VILLARREAL GÓMEZ, Ana, E-03206 Elche (Alicante) (ES)
(74) Representative: ZBM Patents - Zea, Barlocci & Markvardsen
(86) International application number: PCT/EP2012/056035
(87) International publication number: WO 2012/136639

(56) References cited:
- EP-A1- 0 925 795
- WO-A1-02/09784
- DATABASE WPI Week 199228 Thomson Scientific, London, GB; AN 1992-232006 XP002640444, & JP 4 159382 A (SEKISUI AIKO KK) 2 June 1992 (1992-06-02)
- DATABASE WPI Week 198832 Thomson Scientific, London, GB; AN 1988-222376 XP002640445, & JP 63 128089 A (KOATSU GAS KOGYO KK) 31 May 1988 (1988-05-31)

## Description

The present invention relates to the field of adhesives and uses thereof.

### BACKGROUND ART

Attempts to replace traditional sutures in the field of surgery by adhesive joining techniques have been numerous. The purpose is multiple, an attempt to save time in sealing wounds, an attempt to avoid the traditional traumatism entailed in sutures, and finally a more uniform tissue joint, where the pulling forces that tend to separate the joined areas are homogeneously distributed.

Basically, the adhesives used in medicine, also known as bioadhesives, comprised in the state of the art comprise either one or two compounds.

The compound of the adhesive compositions comprising a single compound is the cyanoacrylic (Adam *et al.,* 2008), with high adhesion properties, and which polymerises rapidly in the presence of water and bases. However, cyanoacrylate adhesives present serious drawbacks, polymerisation is an exothermic reaction that can lead to high temperature and the reticulated polymer is very stiff, which may cause ulceration of the surrounding tissue. And, moreover, the reticulated polymer requires at least two months to get released from the tissue.

The other group of adhesives used for biomedical applications are those comprising two compounds, namely, mixtures of alkyl cyanoacrylates and alkyl carboxyacrylates (see EP0925795); and also, mixtures of alkyl cyanoacrylates and (hydroxy-), (cycloalkyl-)acrylates (see WO02/09784).

The adhesive compositions of alkyl cyanoacrylates and alkyl carboxyacrylates have the advantage that the alkyl carboxyacrylates reduce the rigidity of the cyanoacrylates, producing very strong adhesive joints reducing the time to eliminate the polymerised product, which is less than one month and also reducing the exothermic of the polymerisation reaction. However, they also have the disadvantage that the reticulated adhesive is too stiff and the mixture composition needs to be prepared during clinical application.

Regarding the adhesive compositions comprising alkyl cyanoacrylates and acrylates, they have the advantage over the previous ones that the resulting reticulated adhesives are not too rigid, so that the tissues surrounding the adhesive joint do not suffer from ulcer-type lesions; but, on the other hand, the adhesion capacity of the composition is lower; and as in the other adhesive compositions, the mixture needs to be prepared during clinical application.

In summary, the addition of either alkyl carboxyacrylates or acrylates to alkyl cyanoacrylates, reduces the high exothermic polymerisation reaction of the alkyl cyanoacrylate polymerisation reaction, since said compounds do not polymerise with the alkyl cyanoacrylate.

However, although by adding the acrylates to the alkyl cyanoacrylates, the resulting mixture is less stiff and, hence, it can be used for sealing injuries, the adhesion of the alkyl cyanoacrylates is compromised. And moreover, although the addition of the carboxyacrylates to the alkyl cyanoacrylates reduces the adhesion of the alkyl cyanoacrylate to a less extent, the adhesive mixture cannot be used for sealing injuries. In summary, only either stiff and stronger adhesives or sealants and less strong adhesives are currently available.

An additional limitation to the above mentioned ones of the currently available adhesive compositions comprising two compounds is that the compounds have to be stored separately until their clinical application.

In summary, the aim of the present invention is to get over the failures and shortcoming of the adhesive compositions available in the current state of the art, by providing the new one of the present invention.

### SUMMARY OF THE INVENTION

An aspect of the present invention relates to a novel composition, more particularly to a composition comprising the following compounds: i) a compound of formula (I), 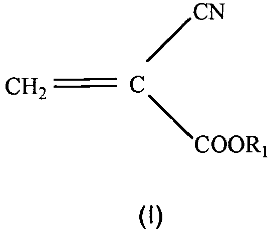 where, R₁ is a radical selected from the group consisting of: (C₁-C₈)-alkyl and R₂OR₃; R₂ is a (C₁-C₄)-alkenyl bi-radical; and, R₃ is a radical selected from the group consisting of: (C₁-C₄)-alkyl; phenyl; and phenyl substituted by a (C₁-C₄)-alkyl;
ii) a compound of formula (II), 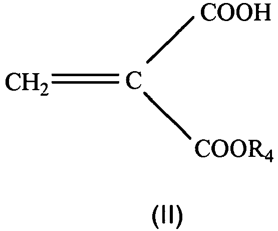 where, R₄ is a radical selected from the group consisting of: (C₁-C₁₀)-alkyl; (C₁-C₁₀)-alkyl substituted by one or two hydroxyls: CH₂-(C₁-C₉)-alkyl wherein one or two carbons of the (C₁-C₉) portion is a CO radical; phenyl; phenyl substituted by a hydroxyl and by a CHO; and phenyl substituted by two radicals independently selected from the group consisting of: (C₁-C₄)-alkyl and hydroxyl;
iii) a compound of formula (III), 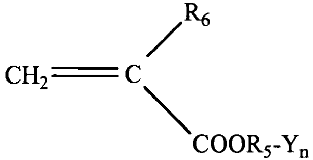 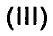 where, R₅ is a bi-radical selected from the group consisting of: (C₁-C₁₀)-alkyl and (C₁-C₁₀)-alkyl substituted by a hydroxyl; R₆ is a radical selected from the group consisting of: hydrogen and (C₁-C₅)-alkyl; n is an integer from 0 to 1, with the proviso that: i) when R₅ is hydrogen, then n is 0, and ii) when R₆ is (C₁-C₅)-alkyl, then R₅ is hydrogen and n is 0; Y is a radical selected from the group consisting of: phenyl; (C₃-C₆)-cycloalkyl; phenyl substituted by a radical selected from the group consisting of: OR₆, COOR₇, CONHR₈, CN and hydroxyl; (C₃-C₆)-cycloalkyl substituted by a radical selected from the group consisting of: OR₇, COOR₈, CONHR₉, CN and hydroxyl; and OR₁₀; R₇ is a (C₁-C₃)-alkyl; R₈ and R₉ are radicals independently selected from the group consisting of (C₁-C₃)-akyl and hydrogen; and R₁₀ is a radical selected from the group consisting of: hydrogen and acryloyl.

The composition of the present invention has the advantage that the three compounds can be mixed together up to 3 weeks, so that there is no need to keep the three compounds separately. Accordingly, the composition does not need to be prepared during clinical application.

Additionally, the composition of the present invention has a surprisingly increased adhesive capacity over those available in the state of the art.

Furthermore, the composition of the present invention has the further advantages that it works efficiently on tissues with a greasy skin (e.g. human ones), as well as on surfaces subjected to relatively significant tensile strengths, such as the skin of the elbow or the knee.

Another aspect of the present invention relates to the composition of the present invention for use in medicine and veterinary.

Furthermore, another aspect of the present invention is the composition of the present invention for use as bioadhesive.

### BRIEF DESCRIPTION OF THE DRAWINGS

FIG. 1 and 2 IR spectra of a ethyl-2-cyanoacrylate:6-hydroxyhexyl acrylate: ethyl-2-carboxyacrylate composition in the proportions 80:15:5 (vol%) stored at 25°C, (a) 5 minutes and (b) 3 weeks after preparation of the mixture, respectively (wherein t=time; w= weeks; min=minutes; T=temperature; Abs=Absorbance; Wn= Wavenumber).
FIG. 3 and 4 IR spectra of a ethyl-2-cyanoacrylate:6-hydroxyhexyl acrylate composition in the proportions 80:20 (vol%), stored at 25°C, (a) 5 minutes and (b) 3 weeks after preparation of the mixture, respectively (wherein t=time; w= weeks; min=minutes; T=temperature; ABS=Absorbance; Wn= Wavenumber).

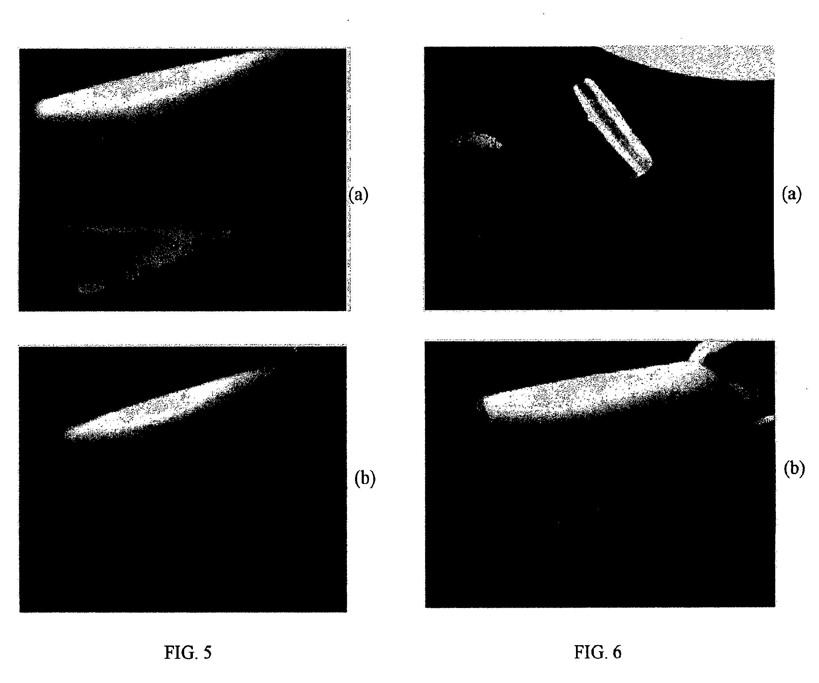

FIG. 5 Image showing the viscosity of a ethyl-2-cyanoacrylate:6-hydroxyhexyl acrylate:ethyl-2-carboxyacrylate composition in the proportions 80:15:5 (vol%) stored at T=25°C, (a) 5 minutes and (b) 3 weeks after the preparation of the mixture.
FIG. 6 Image showing the viscosity of a ethyl-2-cyanoacrylate:6-hydroxyhexyl acrylate composition in the proportions 80:20 (vol%), stored at T=25°C, (a) 5 minutes and (b) 3 weeks after the preparation of the mixture.

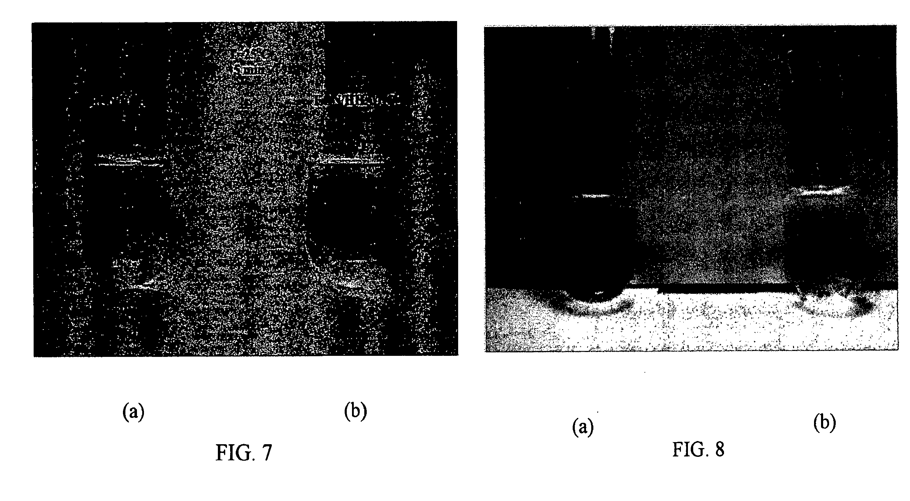

FIG. 7 Image showing the colour of a ethyl-2-cyanoacrylate:6-hydroxyhexyl acrylate:ethyl-2-carboxyacrylate composition in the proportions 80:15:5 (vol%) stored at T=25°C, (a) 5 minutes and (b) 3 weeks after the preparation of the mixture.
FIG. 8 Image showing the colour of a ethyl-2-cyanoacrylate:6-hydroxyhexyl acrylate composition in the proportions 80:20 (vol%), stored at T=25 °C, (a) 5 minutes and (b) 3 weeks after the preparation of the mixture.

### Definitions

All terms used herein should be understood in their ordinary meaning in the art. Specific definitions for certain terms are set forth below and, said definitions should be applied through the specification and claims.

The term "filler" refers to a material added to the adhesive composition to increase their mechanical, rheological and viscoelastic properties.

The term "silica" refers to inorganic materials composed by tetrahedral SiO₄ units repeated in a tridimensional network, which are obtained by precipitation of silicates (also known as precipitated silicas) or by flame reaction of SiCl₄ in an atmosphere of hydrogen and oxygen (also known as fumed silicas).

The expression "carbon black" relates to materials obtained by incomplete combustion of soot, with a structure similar to that of graphite but in a chaotic structure (turbostratic structure). They are mainly constituted by carbon with some heteroatoms, (e.g. oxygen and nitrogen)

The term (C₁-C₄)-alkyl refers to a saturated branched or linear alkyl chain which contains from 1 to 4 carbons. Examples include methyl, ethyl, propyl, isopropyl, butyl, isobutyl, sec-butyl and tert-butyl.

The term (C₁-C₈)-alkyl refers to a saturated branched or linear alkyl chain which contains from 1 to 8 carbons. Examples include methyl, ethyl, propyl, isopropyl, butyl, isobutyl, sec-butyl, tert-butyl, pentyl, hexyl, heptyl and octyl.

The term (C₁-C₁₀)-alkyl refers to a saturated branched or linear alkyl chain which contains from 1 to 10 carbons. Examples include methyl, ethyl, propyl, isopropyl, butyl, isobutyl, sec-butyl, tert-butyl, pentyl, hexyl, octyl, nonyl and decyl.

The term "stabiliser" refers to a chemical that inhibits the formation of free radicals and anionic polymerisation reaction.

### DETAILED DESCRIPTION OF THE INVENTION

The present invention overcomes all the aforementioned limitations by providing novel adhesive compositions.

The composition of the invention comprises the compounds of formula (I), (II) and (III) as detailed above.

The compounds of formula (I) (ECN) are known compounds that can be prepared by conventional methods well known by the skilled person, e.g. see Pascual-Sánchez et al. (see page 1070, Experimental Materials, to page 1071, third paragraph, where the synthesis of alkyl cyanoacrylates is explained).

In a preferred embodiment, the compounds of formula (I) are those wherein R₁ is a (C₁-C₈)-alkyl radical; in a still more preferred embodiment, R₁ is a radical selected from methyl, ethyl, n-butyl, isobutyl, n-hexyl and n-octyl, also known as methyl-2-cyanoacrylate, ethyl-2-cyanoacrylate, n-butyl-2-cyanoacrylate, isobutyl-2-cyanoacrylate, n-hexyl-2-cyanoacrylate and n-octyl-2-cyanoacrylate, respectively.

The compounds of formula (II) (ECA) are known compounds that can be prepared by conventional methods well known by the skilled person, i.e. by esterification of the cyanoacrylic acid with the corresponding alcohol (see EP0925795, page 4, paragraphs 27 and 28).

In a preferred embodiment, the compounds of formula (II) are those wherein R₄ is a (C₁-C₄)-alkyl radical. In a still more preferred embodiment, R₄ is ethyl, compound also known as ethyl-2-carboxyacrylate.

The compounds of formula (III) (HHA) are known compounds that can be prepared by conventional methods well known by the skilled person (i.e. as detailed e.g. in WO02/09784, page 7, lines 7-10).

In a preferred embodiment, the compounds of formula (III) are those wherein: R₅ is a (C₁-C₁₀)-alkyl and n is 0; R₅ is a (C₁-C₁₀)-alkyl substituted by a hydroxyl and n is 0; R₅ is hydrogen and n is 0; and R₆ is a (C₁-C₅)-alkyl, R₅ is hydrogen and n is 0. In a still more preferred embodiment, the compound of formula (III) is selected from the group consisting of methyl methacrylate, 6-hydroxyhexyl acrylate and acrylic acid.

Regarding the specific proportions of the three compounds of the composition of the present invention, they can vary within a relatively broad interval, depending on the application for which the composition is used. The proportions of the three compounds are defined by the ratio X:Y:(100-X-Y), wherein X represents the parts of the compound of formula (I), and is between 60 and 90 parts per volume, both inclusive; Y represents the parts of the compound of formula (II) and is between 1 and 50 parts per volume, both inclusive; and (100-X-Y) represents the parts per volume of the compound of formula (III).

In a preferred embodiment, the proportions in volume per cent (vol.%) of the three compounds of formula (I), (II) and (III) (ECN:ECA:HHA) of the composition of the present invention are (60-90):(5-15):(5-25), respectively, being the sum of compounds equal to 100. In a more preferred embodiment, the compounds of formula (I), (II) and (III) (ECN:ECA:HHA) are mixed, in volume per cent (vol%) in the following proportions 90:5:5, respectively. In a still more preferred embodiment of the invention, the compounds of formula (I), (II) and (III) (ECN:ECA:HHA) are mixed, in volume per cent, in the following proportions 80:5:15, respectively. In a still more preferred embodiment of the invention, the compounds of formula (I), (II) and (III) (ECN:ECA:HHA) are mixed, in volume per cent, in the following proportions 70:10:20, respectively.

Particularly, preferred compositions are the following ones (in vol%): 90 ethyl-2-cyanoacrylate:5 ECA:5 HHA; 80 ethyl-2-cyanoacrylate:5 ECA:15 HHA; 70 ethyl-2-cyanoacrylate:10 ECA:30 HHA; 90 n-octyl-2-cyanoacrylate:5 ECA:5 HHA; 80 n-octyl-2-cyanoacrylate:5 ECA:15 HHA; and 70 n-octyl-2-cyanoacrylate:10 ECA:20 HHA; and (75-90) n-butyl-2-cyanoacrylate:(5-15) ECA:(5-15) HHA.

The composition of the present invention may further comprise a filler, at least one stabiliser, a dye, or any combination thereof. In a preferred embodiment, the filler is selected from silica and carbon black. In a preferred embodiment, the filler is in an amount between 0.5 and 10 weight per cent (wt%) with respect to the total weight of the adhesive composition of the present invention. Preferably, the stabiliser is selected from hydroquinone, p-toluene sulphonic acid, sulfur dioxide and diphosphorus pentoxide. In a preferred embodiment, the stabiliser is in an amount between 10⁻² and 2 wt% with respect to the total weight of the adhesive composition of the present invention. The filler as well as the stabiliser(s) adjust viscosity and mechanical properties of the adhesive composition of the present invention.

As already mentioned above, the compound of formula (I) reduces the exothermic polymerisation reaction of the ECN as well as the rigidity of the ECN. However, the disadvantage of the adhesive compositions disclosed in the state of the art, comprising either alkyl carboxyacrylates or acrylates, and alkyl cyanoacrylates is that the addition of or reduces the adhesive capacity of the alkyl cyanoacrylate. By mixing the three compounds of formula (I), (II) and (III) disclosed in the present invention, the inventors have surprisingly found that the adhesive capacity of the resulting composition is increased (see data in tables 1 and 2 below).

Using different proportions of the three compounds, in the above mentioned suitable ranges, a modulation of the adhesive and sealing properties of the adhesive mixture are obtained, particularly: (i) the strength of the adhesive joint is reinforced, or on the contrary reduced; and, (i) the mechanical rigidity of the polymerised composition is increased or decreased.

In summary, the composition of the invention combines enough tensile strength and softness in the hardened product.

Additionally, the composition disclosed herein have optimal transparency in the polymerised product, show no tissue toxicity, adhere at room temperature (body temperature) and in the presence of fat, and the polymerised product has an acceptable elimination time.

Advantageously, the three compounds of the composition of the present invention do not need to be stored in independent containers. The composition of the present invention is chemically stable on time (see example 1). Furthermore, the stability of the disclosed composition is also supported by the fact that its viscosity and colour are also stable on time (see example 2).

The three compounds can be mixed together and stored protected against exposure to light and moisture, the temperature range for storage being between 6 and 8°C.

The composition of the present invention is stable for 3 weeks at room temperature, after being mixed and before being applied on living tissues. It has been verified that the adhesive capacity reduces, and with it, the strength of the joining of tissues after one hour of being applied. The anionic polymerisation of the three monomers that constitute the mixture is produced due to the -OH groups derived from water or humidity. The miscibility of the three compounds used in the composition of the present invention is optimal, so that a single phase is achieved.

Finally, another aspect of the present invention is the use of the composition of the present invention for the manufacture of a bioadhesive composition for surgery, preferably, surgery by adhesive joining techniques.

Throughout the description and claims the word "comprise" and variations of the word, are not intended to exclude other technical features, additives, components, or steps. Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

### EXAMPLES

### Example 1 Stability of ECN:HHA:ECA composition versus ECN:HHA composition

Eighty parts per volume of ethyl-2-cyanoacrylate, 15 parts per volume of 6-hydroxyhexyl acrylate and 5 parts per volume of ethyl-2-carboxyacrylate were mixed and kept at 25°C for 3 weeks. Similarly, 80 parts per volume of ethyl cyanoacrylate and 20 parts per volume of 6-hydroxyhexyl acrylate were mixed and kept at 25°C for 3 weeks. Two samples were taken from each mixture, one after 5 minutes and one after 3 weeks of the preparation of the adhesive mixtures.

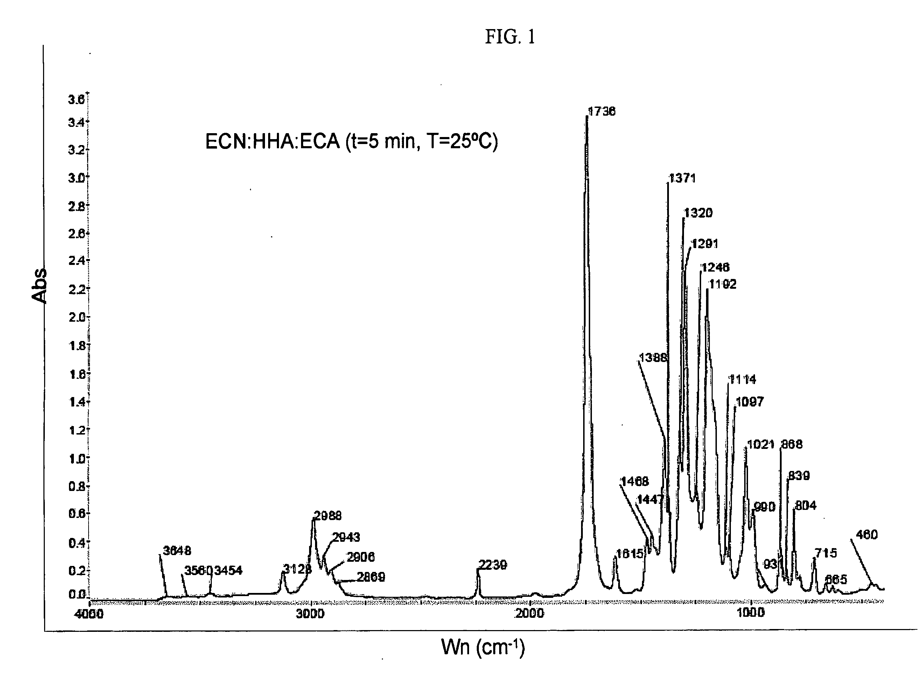

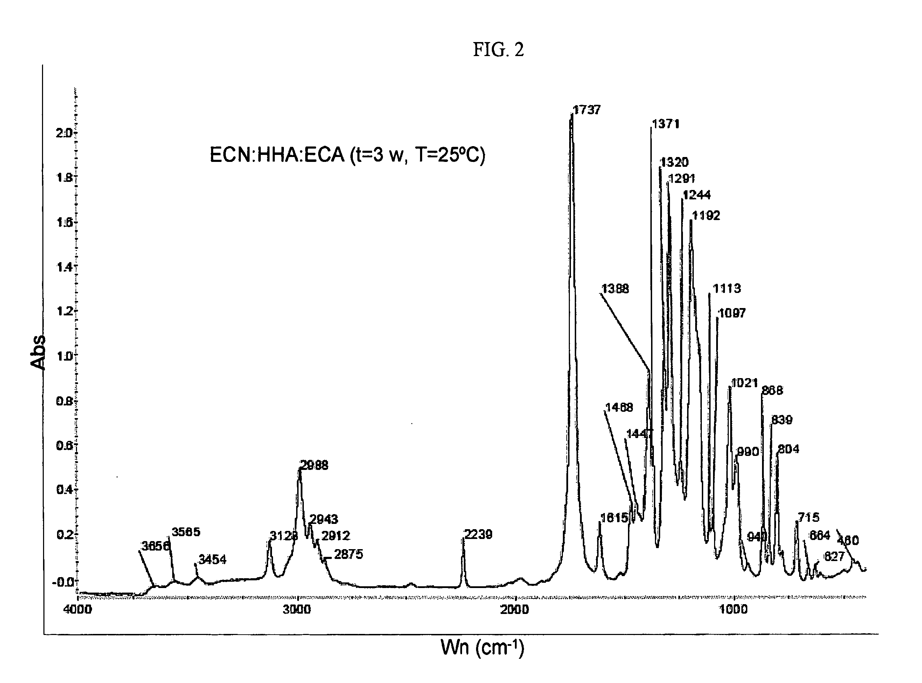

IR spectra were carried out for each sample of each of the two tested compositions. The IR spectrum of the sample taken after 3 weeks of the preparation of the composition comprising the three compounds did not change with respect to the IR spectrum of the sample taken after 5 minutes (see figures 1 and 2).

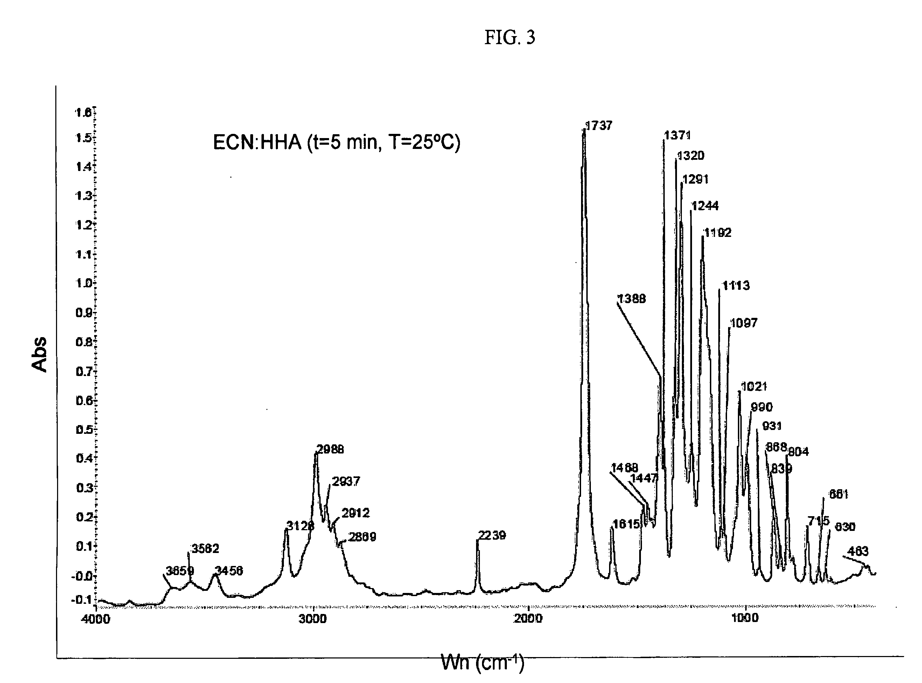

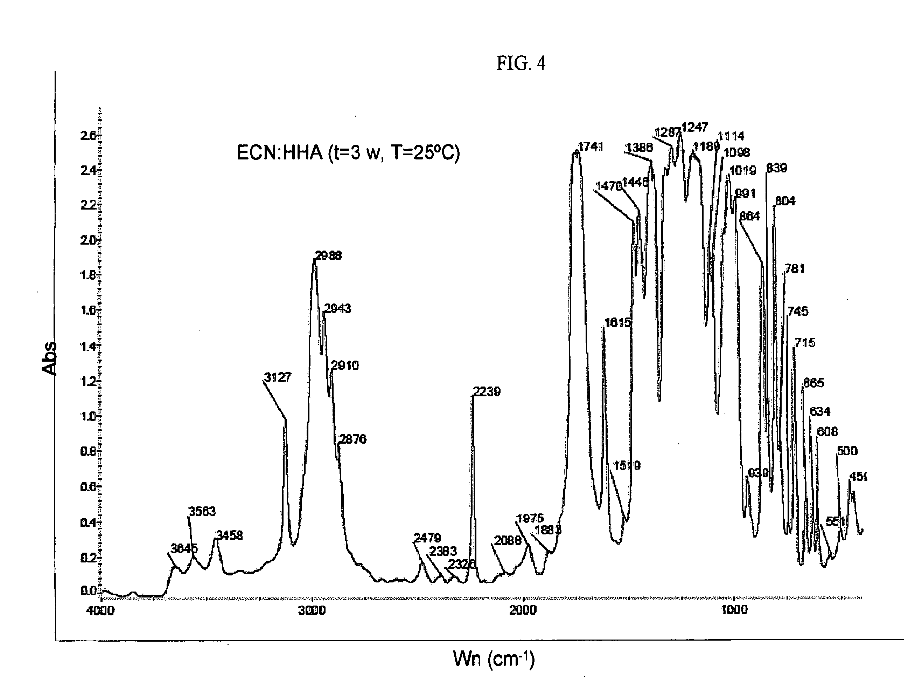

On the contrary, the IR spectrum of the sample taken after 3 weeks of the preparation of the composition comprising the two compounds was not stable on time, as shown when comparing it with the IR spectra obtained after 5 minutes and 3 weeks of the preparation of the mixture (see figures 3 and 4).

### Example 2 Viscosity and colour of the ECN:HHA:ECA composition versus the ECN:HHA composition

Eighty parts per volume of ethyl-2-cyanoacrylate, 15 parts per volume of 6-hydroxyhexyl acrylate and 5 parts per volume of ethyl-2-carboxyacrylate were mixed and kept at 25°C for 3 weeks. Similarly, 80 parts per volume of ethyl-2-cyanoacrylate and 20 parts per volume of 6-hydroxyhexyl acrylate were mixed and kept at 25°C for 3 weeks. Two samples were taken from each mixture, one after 5 minutes and one after 3 weeks of the preparation of the adhesive mixtures.

The colour and viscosity of the samples taken from each of the above compositions were visually tested. The viscosity and colour of the composition comprising the three compounds mentioned above did not change after 3 weeks of the preparation. On the contrary, in the case of the sample of the composition comprising the two compounds, after 3 weeks of storage, a yellowish viscous gel was formed.

### Example 3 Assays to assess the adhesion capacity of the adhesive composition of the invention

To assess the strength of the adhesive joints generated by the invention's adhesive compositions, in particular, ethyl-2-cyanoacrylate, ethyl-2-carboxyacrylate and 6-hydroxyhexyl acrylate in different proportions, a protocol for adhesion to two types of surfaces, namely, biological material of a dead animal (pork skin) and synthetic material (polyvinyl chloride or PVC) was carried out.

### Adhesive joints with pork skin

The test was carried out on a sample of pork skin, which was chosen since it is the most similar skin to the human one and, moreover, has the inconvenience of being greasy, which is an important antiadhesion factor. The skin of the pork was obtained from the legs. From the skin sample, pieces of 60x30mm were cut, and soaked in saline solution for 5 minutes. Then, the skin pieces were dried using filter paper and allowed to air dry onto a polypropylene surface. An amount of 0.04 ml of the composition was applied onto the two pork skin pieces to test, and extended on a surface of 30x15 mm of each of the two pork skins tested. The assays were carried out 45 seconds afterwards. The test was finished once the adhesive joint was broken or the adhesive joint was stronger than the load cell of the apparatus used to test it.

The skin joints were separated by single lap-shear assay. A TA-XT2i texture analyser was used. The pulling rate of the assay was 0.5 mm/s and the speed of the data acquisition was 200 data/sec. Jaw clamps were used to hold the test samples during the separation of the joints. Table 1 below summarises the adhesive strength values obtained for pork skin/adhesive/pork skin joints, wherein the three compounds (in different proportions) of the compositions tested were ethyl-2-cyanoacrylate, ethyl-2-carboxyacrylate and 6-hydroxyhexyl acrylate.

**Table 1:**

| ECN (vol%) | ECA (vol%) | HHA (vol%) | Adhesive strength (KPa) | Locus of failure |
|---|---|---|---|---|
| 70 | 20 | 10 | 77.4 | mixed |
| 70 | 30 | 0 | 72.5 | mixed |
| 70 | 0 | 30 | 70.8 | mixed |
| 60 | 30 | 10 | 40.0 | mixed |
| 60 | 40 | 0 | 15.3 | mixed |
| 60 | 0 | 40 | 3.4 | mixed |

wherein KPa stands for KiloPascal; and "mixed" means that the failure of the joint was both adhesion, i.e. separation of the adhesive from the pork skin and cohesive failure in the adhesive, this later one found dominant-).

As shown in Table 1, for the same amount of the adhesive component (ECN), the adhesion of the three compounds mixture is increased compared to only ECA or HHA.

### Adhesive joints with PVC

Rectangular test samples of rigid PVC of 30x150 mm were used. The test samples were cleaned with methyl ethyl acetone. After 30 minutes, an amount of 0.06 ml of the tested adhesive composition was applied onto the surface of one of the two test samples used for each assay; the test sample with the composition was subsequently joined to another test sample at 2 kg/m² of pressure. The adhesive joint had an area of 35x35 mm. The single lap-shear assays were carried out 72 hours afterwards.

The assays with PVC were carried out using the universal testing machine (e.g. INSTRON 4411 machine). The pulling rate during the assay was 100 mm/min.

Table 2 below shows the adhesive strength values obtained for PVC/adhesive/PVC joints, wherein the three compounds (in different proportions) of the compositions tested were ethyl-2-cyanoacrylate, ethyl-2-carboxyacrylate and 6-hydroxyhexyl acrylate.

**Table 2**

| ECN (vol%) | ECA (vol%) | HHA (vol%) | Adhesive strength (KPa) | Locus of failure |
|---|---|---|---|---|
| 70 | 30 | 0 | 85.1 | type 1 |
| 70 | 20 | 10 | 139.4 | type 1 |
| 70 | 15 | 15 | 100.2 | type 1 |
| 70 | 10 | 20 | 128.5 | type 1 |
| 80 | 20 | 0 | 108 | type 1 |
| 80 | 10 | 10 | 200 | type 1 |
| 80 | 15 | 5 | 159.3 | type 1 |
| 80 | 5 | 15 | 193.2 | type 1 |

| | | | | |
|---|---|---|---|---|
| ECN, ECA and HHA stand for alkyl carboxyacrylate, carboxyacrylate, and acrylate, respectively; and type 1 means that the failure was fully cohesive in the adhesive. | | | | |

As shown in Table 2, for the same amount of the adhesive component (ECN), the adhesion of the three compounds mixture is increased compared to only ECA or HHA.

### REFERENCES CITED IN THE APPLICATION

Adam et al. "The cyanoacrylate topical skin adhesives", American Journal of Emergency Medicine, 2008, 26, 490-496.
Pascual-Sánchez et al. "Synthesis and characterization of a new acrylic adhesive mixture for use in ocular strabismus surgery", The Journal of Adhesion, 2003, 69, 1067-1089.

## Claims

1. A composition comprising the following compounds:
i) a compound of formula (I), 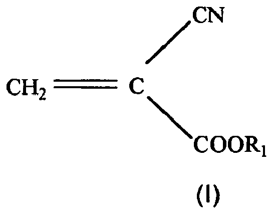 wherein:
R₁ is a radical selected from the group consisting of: (C₁-C₈)-alkyl and R₂OR₃;
R₂ is a (C₁-C₄)-alkenyl bi-radical; and
R₃ is a radical selected from the group consisting of: (C₁-C₄)-alkyl, phenyl; and phenyl substituted by a (C₁-C₄)-alkyl;
ii) a compound of formula (II), 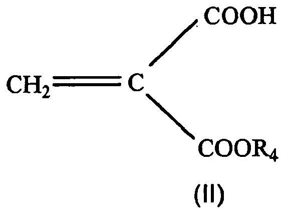 wherein:
R₄ is a radical selected from the group consisting of: (C₁-C₁₀)-alkyl; (C₁-C₁₀)-alkyl substituted by one or two hydroxyls; CH₂-(C₁-C₉)-alkyl, wherein one or two carbons of the (C₁-C₉) portion is a CO radical; phenyl; phenyl substituted by a hydroxyl and by a CHO; and phenyl substituted by two radicals independently selected from the group consisting of: (C₁-C₄)-alkyl and hydroxyl.
iii) a compound of formula (III), 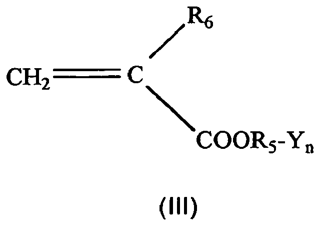 wherein:
R₅ is a bi-radical selected from the group consisting of: (C₁-C₁₀)-alkyl; (C₁-C₁₀)-alkyl substituted by a hydroxyl;
R₆ is a radical selected from the group consisting of hydrogen and (C₁-C₅)-alkyl;
n is an integer from 0 to 1, with the proviso that: i) when R₅ is hydrogen, then n is 0; and ii) when R₆ is (C₁-C₅)-alkyl, then R₅ is hydrogen and n is 0;
Y is a radical selected from the group consisting of: phenyl; (C₃-C₆)-cycloalkyl; phenyl substituted by a radical selected from the group consisting of: OR₆, COOR₇, CONHR₈, CN and hydroxyl; (C₃-C₆)-cycloalkyl substituted by a radical selected from the group consisting of: OR₇, COOR₈, CONHR₉, CN and hydroxyl; and OR₁₀;
R₇ is a (C₁-C₃)-alkyl;
R₈ and R₉ are radicals independently selected from the group consisting of (C₁-C₃)-akyl and hydrogen; and
R₁₀ is a radical selected from the group consisting of: hydrogen and acryloyl.

2. The composition of claim 1, wherein the compound of formula (I) is selected from the group consisting of methyl-2-cyanoacrylate, ethyl-2-cyanoacrylate, n-butyl-2-cyanoacrylate, isobutyl-2-cyanoacrylate, n-hexyl-2-cyanoacrylate and n-octyl-2-cyanoacrylate.

3. The composition of any of the claims 1-2, wherein the compound of formula (II) is ethyl-2-carboxyacrylate.

4. The composition of any of the claims 1-3, wherein the compound of formula (III) is selected from the group consisting of 6-hydroxyhexyl acrylate, methyl methacrylate and acrylic acid.

5. The composition of any of the claims 1-4, wherein the proportion in volume per cent of each of the compounds of formula (I), (II) and (III) is in the range of (70-90), (5-15) and (5-25), respectively, being the sum of three compounds equal to 100.

6. The composition of claim 5, wherein the proportion in volume per cent of each of the compounds of formula (I), (II) and (III) is 90, 5 and 5, respectively.

7. The composition of claim 5, wherein the proportion in volume per cent of each of the compounds of formula (I), (II) and (III) is 80, 5 and 15, respectively.

8. The composition of claim 5, wherein the proportion in volume per cent of each of the compounds of formula (I), (II) and (III) is 70, 10 and 20, respectively.

9. The composition of any of the claims 1-8, further comprising a filler, at least one stabiliser, a dye, or any combination thereof.

10. The composition of claim 9, wherein the filler is selected from the group consisting of silica and carbon black.

11. The composition of any of the claims 9 and 10, wherein the filler is in an amount between 0.5 and 10 weight per cent of the total weight of the composition of any of the claims 1-8.

12. The composition of any of the claims 9-11, wherein the at least one stabiliser is selected from the group consisting of hydroquinone, p-toluene sulphonic acid, sulfur dioxide, and diphosphorus pentoxide.

13. The composition of claim 12, wherein the at least one stabiliser is in an amount between 10⁻² and 2 weight per cent with respect to the total weight of the composition of any of the claims 1-8.

14. A composition as defined in any of the claims 1-13 for use in medicine and veterinary.

15. A composition as defined in any of the claims 1-13, for use as bioadhesive.

## Patentansprüche

1. Eine Zusammensetzung umfassend die folgenden Verbindungen:
i) eine Verbindung der Formel (I), 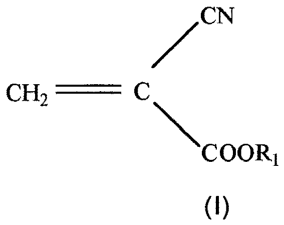 worin:
R₁ ein Radikal ausgewählt aus der Gruppe bestehend aus: (C₁-C₈)-Alkyl und R₂OR₃ ist;
R₂ ein (C₁-C₄)-Alkenyl Biradical ist; und
R₃ ein Radikal ausgewählt aus der Gruppe bestehend aus: (C₁-C₄)-Alkyl; Phenyl; und
durch ein (C₁-C₄)-Alkyl substituiertes Phenyl ist;
ii) eine Verbindung der Formel (II), 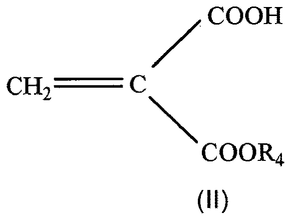 worin:
R₄ ein Radikal ausgewählt ist aus der Gruppe bestehend aus: (C₁-C₁₀)-Alkyl; durch ein oder zwei Hydroxylgruppen substituiertes (C₁-C₁₀)-Alkyl; CH₂(C₁-C₉)-Alkyl, worin ein oder zwei Kohlenstoffatome des (C₁-C₉)-Teils ein CO-Radikal ist; Phenyl; durch ein Hydroxyl und durch ein CHO substituiertes Phenyl; und Phenyl, das durch zwei Radikale substituiert ist, die unabhängig voneinander ausgewählt aus der Gruppe bestehend aus: (C₁-C₄)-Alkyl und Hydroxyl sind;
iii) eine Verbindung der Formel (III), 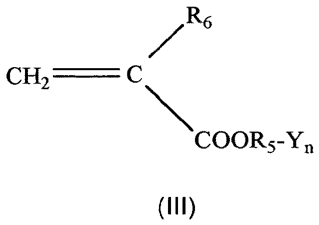 worin:
R₅ ein Biradikal ausgewählt aus der Gruppe bestehend aus: (C₁-C₁₀)-Alkyl; durch ein Hydroxyl substituiertes (C₁-C₁₀)-Alkyl ist;
R₆ ein Radikal ausgewählt aus der Gruppe bestehend aus Wasserstoff und (C₁-C₅)-Alkyl ist;
n eine Ganzzahl von 0 bis 1 ist, mit der Maßgabe, dass: i) wenn R₅ Wasserstoff ist,
dann ist n = 0; und ii) wenn R₆ (C₁-C₅)-Alkyl ist, dann ist R₅ Wasserstoff und n = 0;
Y ein Radikal ist, das ausgewählt ist aus der Gruppe bestehend aus: Phenyl; (C₃-C₆)-Cycloalkyl; Phenyl, das durch ein Radikal substituiert ist, das ausgewählt ist aus der Gruppe bestehend aus: OR₆, COOR₇, CONHR₈, CN und Hydroxyl; (C₃-C₆)-Cycloalkyl, das durch ein Radikal substituiert ist, das ausgewählt aus der Gruppe bestehend aus: OR₇, COOR₈, CONHR₉, CN und Hydroxyl; und OR₁₀ ist;
R₇ ein (C₁-C₃)-Alkyl ist:
R₈ und R₉ Radikale sind, die unabhängig voneinander ausgewählt aus der Gruppe bestehend aus (C₁-C₃)-Alkyl und Wasserstoff sind; und
R₁₀ ein Radikal ausgewählt aus der Gruppe bestehend aus: Wasserstoff und Acryloyl ist.

2. Die Zusammensetzung des Anspruchs 1, wobei die Verbindung der Formel (I) ausgewählt aus der Gruppe bestehend aus Methyl-2-cyanoacrylat, Ethyl-2-cyanoacrylat, n-Butyl-2-cyanoacrylat, Isobutyl-2-cyanoacrylat, n-Hexyl-2-cyanoacrylat und n-Octyl-2-cyanoacrylat ist.

3. Die Zusammensetzung von einem der Ansprüche 1-2, wobei die Verbindung der Formel (II) Ethyl-2-carboxyacrylat ist.

4. Die Zusammensetzung von einem der Ansprüche 1-3, wobei die Verbindung der Formel (III) ausgewählt aus der Gruppe bestehend aus 6-Hydroxyhexylacrylat, Methylmethacrylat und Acrylsäure ist.

5. Die Zusammensetzung von einem der Ansprüche 1-4, wobei das prozentige Volumenverhältnis von jeder der Verbindungen der Formel (I), (II) und (III) jeweils in dem Bereich von (70-90), (5-15) und (5-25) ist, wobei die Summe der drei Verbindungen 100 gleicht.

6. Die Zusammensetzung des Anspruchs 5, wobei das prozentige Volumenverhältnis von jeder der Verbindungen der Formel (I), (II) und (III) jeweils 90, 5 und 5 ist.

7. Die Zusammensetzung des Anspruchs 5, wobei das prozentige Volumenverhältnis von jeder der Verbindungen der Formel (I), (II) und (III) jeweils 80, 5 und 15 ist.

8. Die Zusammensetzung des Anspruchs 5, wobei das prozentige Volumenverhältnis von jeder der Verbindungen der Formel (I), (II) und (III) jeweils 70, 10 und 20 ist.

9. Die Zusammensetzung von einem der Ansprüche 1-8, weiterhin umfassend einen Füllstoff, mindestens einen Stabilisator, einen Farbstoff, oder eine Kombination davon.

10. Die Zusammensetzung des Anspruchs 9, wobei der Füllstoff ausgewählt aus der Gruppe bestehend aus Siliciumdioxid und Ruß ist.

11. Die Zusammensetzung von einem der Ansprüche 9 und 10, wobei der Füllstoff in einer Menge zwischen 0,5 und 10 Gew.-% bezüglich des Gesamtgewichts der Zusammensetzung von einem der Ansprüche 1-8 ist.

12. Die Zusammensetzung von einem der Ansprüche 9-11, wobei der mindestens eine Stabilisator ausgewählt aus der Gruppe bestehend aus Hydrochinon, p-Toluolsulfonsäure, Schwefeldioxid, und Diphosphorpentoxid ist.

13. Die Zusammensetzung des Anspruchs 12, wobei der mindestens ein Stabilisator in einer Menge zwischen 10⁻² und 2 Gew.-% bezüglich des Gesamtgewichts der Verbindung von einem der Ansprüche 1-8 ist.

14. Eine Zusammensetzung wie in einem der Ansprüche 1-13 definiert zur Verwendung in der Medizin und in der Tierheilkunde.

15. Eine Zusammensetzung wie in einem der Ansprüche 1-13 definiert zur Verwendung als biologischer Klebstoff.

## Revendications

1. Une composition comprenant les composés suivants :
i) un composé de formule (I), 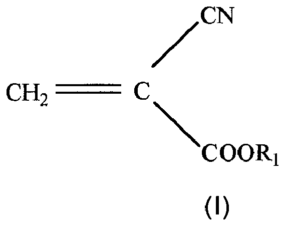 dans lequel :
R₁ est un radical choisi dans le groupe constitué de : alkyle en (C₁-C₈) et R₂OR₃ ;
R₂ est un bi-radical alcényle en (C₁-C₄) ; et
R₃ est un radical choisi dans le groupe constitué de : alkyle en (C₁-C₄); phényle ; et
phényle substitué par un alkyle en (C₁-C₄) ;
ii) un composé de formule (II), 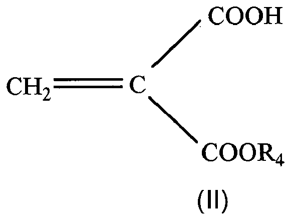 dans lequel :
R₄ est un radical choisi dans le groupe constitué de : alkyle en (C₁-C₁₀) ; alkyle en (C₁-C₁₀) substitué par un ou deux groupes hydroxyle ; alkyle en CH₂(C₁-C₉), dans lequel un ou deux carbones de la portion (C₁-C₉) est un radical CO ; phényle, phényle substitué par un groupe hydroxyle et par un CHO ; et phényle substitué par deux radicaux choisis indépendamment du groupe constitué de : alkyle en (C₁-C₄) et hydroxyle.
iii) un composé de formule (III), 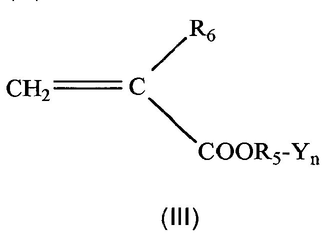 dans lequel :
R₅ est un bi-radical choisi dans le groupe constitué de : alkyle en (C₁-C₁₀) ; alkyle en (C₁-C₁₀) substitué par un hydroxyle ;
R₆ est un radical choisi dans le groupe constitué d'hydrogène et d'alkyle en (C₁-C₅) ;
n est un nombre entier allant de 0 à 1, avec la condition que : i) lorsque R₅ est hydrogène, alors n est 0 ; et ii) lorsque R₆ est alkyle en (C₁-C₅), alors R₅ est hydrogène et n est 0 ;
Y est un radical choisi dans le groupe constitué de : phényle, cycloalkyle en (C₃-C₆) ;
phényle substitué par un radical choisi dans le groupe constitué de : OR₆, COOR₇, CONHR₈, CN et hydroxyle ; cycloalkyle en (C₃-C₆) substitué par un radical choisi dans le groupe constitué de : OR₇, COOR₈, CONHR₉, CN et hydroxyle ; et OR₁₀ ;
R₇ est alkyle en (C₁-C₃) :
R₈ et R₉ sont radicaux indépendamment choisis dans le groupe constitué d'alkyle en (C₁-C₃) et d'hydrogène ; et
R₁₀ est un radical choisi dans le groupe constitué de : hydrogène et acryloyle.

2. La composition de la revendication 1, dans laquelle le composé de formule (I) est choisi dans le groupe constitué de 2-cyanoacrylate de méthyle, 2-cyanoacrylate d'éthyle, 2-cyanoacrylate de n-butyle, 2-cyanoacrylate d'isobutyle, 2-cyanoacrylate de n-héxyle et 2-cyanoacrylate de n-octyle.

3. La composition de l'une quelconque des revendications 1-2, dans laquelle le composé de formule (II) est le 2-carboxyacrylate d'éthyle.

4. La composition de l'une quelconque des revendications 1-3, dans laquelle le composé de formule (III) est choisi dans le groupe constitué de l'acrylate de 6-hydroxyhéxyle, du méthacrylate de méthyle et de l'acide acrylique.

5. La composition de l'une quelconque des revendications 1-4, dans laquelle la proportion en pourcentage en volume de chacun des composés de formule (I), (II) et (III) est dans l'intervalle de (70-90), (5-15) et (5-25), respectivement, étant le total des trois composés égal à 100.

6. La composition de la revendication 5, dans laquelle la proportion en pourcentage en volume de chacun des composés de formule (I), (II) et (III) est 90, 5 et 5, respectivement.

7. La composition de la revendication 5, dans laquelle la proportion en pourcentage en volume de chacun des composés de formule (I), (II) et (III) est 80, 5 et 15, respectivement.

8. La composition de la revendication 5, dans laquelle la proportion en pourcentage en volume de chacun des composés de formule (I), (II) et (III) est 70, 10 et 20, respectivement.

9. La composition de l'une quelconque des revendications 1-8, comprenant en outre une charge, au moins un agent de stabilisation, un colorant, ou une combinaison quelconque de ceux-ci.

10. La composition de la revendication 9, dans laquelle la charge est choisie dans le groupe constitué de silice et de suie.

11. La composition de l'une quelconque des revendications 9 et 10, dans laquelle la charge est en une quantité d'entre 0,5 et 10 pour cent en poids du poids total de la composition de l'une quelconque des revendications 1-8.

12. La composition de l'une quelconque des revendications 9-11, dans laquelle l'au moins un agent de stabilisation est choisi dans le groupe constitué de l'hydroquinone, de l'acide p-toluènesulphonique, du dioxyde de soufre et du pentoxyde de phosphore.

13. La composition de la revendication 12, dans laquelle l'au moins un agent de stabilisation est en une quantité d'entre 10⁻² et 2 pour cent en poids par rapport au poids total de la composition de l'une quelconque des revendications 1-8.

14. Une composition telle que définie dans l'une quelconque des revendications 1-13 pour son utilisation en médecine humaine et vétérinaire.

15. Une composition telle que définie dans l'une quelconque des revendications 1-13 pour son utilisation comme bioadhésif.
